Europäisches Patentamt

European Patent Office

Office européen des brevets

(19)

(11) Publication number: **0 203 107 B1**

(12) **EUROPEAN PATENT SPECIFICATION**

(45) Date of publication of patent specification: **06.11.91**

(21) Application number: **85905615.2**

(22) Date of filing: **22.10.85**

(86) International application number:
**PCT/EP85/00557**

(87) International publication number:
**WO 86/02651 (09.05.86 86/10)**

(51) Int. Cl.⁵: **C07K 15/06, C07K 3/02,
C07K 15/00, C12P 21/00,
G01N 33/574, G01N 33/577,
G01N 33/68, A61K 39/395,
A61K 37/02, //(C12P21/00,
C12R1:91)**

(54) SPECIFIC CARBOHYDRATE-BINDING PROTEINS (LECTINS) OF MAMMALIAN TUMOR CELLS.

(30) Priority: **23.10.84 DE 8411275 U**

(43) Date of publication of application:
**03.12.86 Bulletin 86/49**

(45) Publication of the grant of the patent:
**06.11.91 Bulletin 91/45**

(84) Designated Contracting States:
**AT BE CH DE FR GB IT LI LU NL SE**

(56) References cited:

**CHEMICAL ABSTRACTS, vol. 96, no. 5, 01
Febr 1982, Columbus(Ohio)USA L B GRABEL
et al.: " A fucan-specific lectin on teratocar-
cinoma stem cells", see p. 486, abstract
33150j**

(73) Proprietor: **Max-Planck-Gesellschaft zur För-
derung der Wissenschaften e.V.
Bunsenstrasse 10
W-3400 Göttingen(DE)**

(72) Inventor: **CRAMER, Friedrich
Jakob-Henle-Str. 18
W-3400 Göttingen(DE)**
Inventor: **GABIUS, Hans-Joachim
Hermann-Rhein-Str. 3
W-3400 Göttingen(DE)**

(74) Representative: **Vossius & Partner
Siebertstrasse 4 P.O. Box 86 07 67
W-8000 München 86(DE)**

CHEMICAL ABSTRACTS, vol. 95, no. 15, 12 Oct 1981, Columbus, Ohio, USA A RAZ et al.: "Lectin-like activities associated with human and murine neoplastic cells", see p. 487, abstract 130669x

BIOLOGICAL ABSTRACTS, vol. 77, no. 8, 1984, Philadelphia, PA (USA) C F ROFF et al.: "Endogenous lectins from cultured cells. Isolation and characterization of carbohydrate-binding proteins from 3T3 fibroblasts", see p.6344, column 2, abstr. 57678

BIOLOGICAL ABSTRACTS, vol. 78, no. 10, 1984, Philadelphia, PA (US) H.-J. GABIUS et al.: "Endogenous lectins of bovine pancreas", see p.8335, col. 1, abstr. 74688

BIOLOGICAL ABSTRACTS, vo. 79, no. 9, 1985, Philadelphia, PA, USA H.-J. GABIUS et al.: "Biochemical characterization of endogenous carbohydrate-binding proteins from spontaneous murine rhabdomysacroma mammary adenocarcinoma and ovarian teratoma",see p. AB-564-565, col. 2, abstract 78443

**Description**

This invention relates to carbohydrate-binding proteins (lectins) of mammalian tumor cells which specifically recognize and bind to carbohydrate molecules and to methods of isolating these lectins from mammalian tumor cells.

The designation "lectin" is derived from the property of certain proteins to "select" (i.e. recognize) specific carbohydrate structures and to form a lectin-carbohydrate complex.

Lectins can be defined as follows:

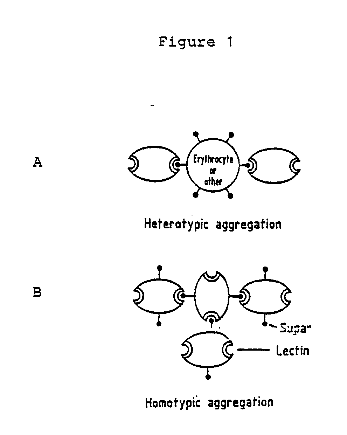

- the recognition of carbohydrates is highly specific and thus comparable to the antigen-specificity of antibodies or the substrate-specificity of enzymes;
- in contrast to antibodies which can also specifically recognize carbohydrate-residues of glycoconjugates, lectins are of non-immune origin;
- in contrast to enzymes which can also specifically recognize carbohydrates or glycoconjugates, lectins do not display any detectable enzymatic activity;
- they display carbohydrate-inhibitable homotypic and heterotypic agglutinating activity (see Fig. 1), e.g. of bacteria or blood cells as trypsinized and glutaraldehyde treated rabbit erythrocytes.

From this definition of lectins it can be taken that for the clear identification of a protein as a lectin, the properties of the protein have to fulfil all the above-mentioned prerequisites. Otherwise the protein in question could, for instance, also be an antibody or an enzyme.

The binding of lectins to their corresponding carbohydrates can be either $Ca^{2+}$-dependent or $Ca^{2+}$-independent, i.e. some of the lectins only form a complex with the respective carbohydrate in the presence of $Ca^{2+}$-ions.

Until very recently lectins were thought to be peculiarities of the plant kingdom. The physiological role of these proteins is still not known. During the last few years it has become apparent that lectins are regular components of almost every cell membrane or cell surface. Although not much is known yet in this field of research, it is suggested that lectins play a key role in many intercellular processes together with the corresponding carbohydrates on other cells. They form what is probably the most important cellular recognition and communication system and might be important in the development of organs, especially in the development of the central nervous system. Furthermore, they are believed to play a role in fertilization (when sperm and egg recognize each other) and are important in endocytosis; see Barondes, Ann.Rev. Biochem. 50 (1981), p. 207.

Gabius et al. (Hoppe-Seyler's Z. Physiol. Chem. 355 (1984), p. 633), describe $Ca^{2+}$-independent lectins which were isolated from bovine pancreas and have a molecular weight of 16,000, 35,000 and 64,000, respectively. They bind specifically the $\beta$-galactosides lactose and asialofetuin and the $\alpha$-galactoside melibiose. Furthermore, fucose-binding lectins which are $Ca^{2+}$-dependent and have a molecular weight of 34,000; 62,000; and 70,000; respectively, are described.

Ashwell et al. (Ann. Rev. Biochem. 51 (1982), p. 531) describe $\beta$-galactoside-specificic receptors of the liver which specifically recognize asialo-glycoside residues of proteins and are responsible for the uptake of these glycoproteins into hepatocytes. Furthermore, a hepatic mannan-specific receptor is described.

Kawasaki et al. (J. Biochem. 88 (1980), p. 1891; J. Biochem. 94 (1983), p. 937) published data of a protein with $Ca^{2+}$-dependent mannan-binding specificity. The protein was isolated from the mesenteric lymph nodes of rats and from human serum. It was, however, not analyzed according to the parameters given above, whether this protein actually is a lectin.

Rutherford et al. (FEBS Lett. 136 (1981), p. 105) describe the isolation and characterization of a mannan-binding lectin of the vitelline membrane of the early chick embryo. The physiological role of this protein, however, is not disclosed.

From a publication of Roberson and Barondes (J. Biol. Chem. 257 (1982), p. 7520) a lectin of Xenopus laevis oocytes, X. laevis embryos and the liver of the adult X. laevis is known. The lectin under investigation displays different specific activities in the three different differentiation stages.

Grabel et al. ( Cell 17 (1979), p. 477) published the occurrence of a carbohydrate-binding component on the surface of teratocarcinoma stem cells. This component is designated by the authors as a lectin-like component and not as a lectin. Thus, this reference does not disclose whether or not the carbohydrate-binding component found is a lectin.

Moreover, this publication does not contain any characterzing data concerning the carbohydrate-binding component. What is disclosed there is just an observation on the association of cells, which can be inhibited by the addition of mannose-rich glycoproteins as yeast invertase, yeast, mannans and horse radish peroxidase. A further publication of Grabel et al. (Biophys., Biochem. Res. Comm. 102 (1981), p.1165) refers to the extraction of mouse teratocarcinoma cells. According to the authors, this extract contains a fucoidan-inhibitable hemagglutination activity.

In the papers of Raz et al. (Cancer Res. 41 (1981), p. 3642), Roche et al. (J. Cell. Biochem. 22 (1983), p. 131) and Teichberg et al. (Proc. Natl. Acad. Sci. USA 72 (1975), p. 1383) a β-galactoside specific hamagglutination activity, a glucose-specific endocytosis activity, and a β-galactoside -specific hemagglutination activity, respectively, are described which were detected on the surface of tumor cells or in the extracts of tumor cells. It has to be understood, however, that the papers of Grabel et al. (supra), Raz et al. (supra), Roche et al. (supra), and Teichberg et al. (supra) do not show the presence of lectins on the surface or in the cytoplasm of tumor cells. The presence of lectins is only proved if all of the above-mentioned parameters characterizing a protein as a lectin are investigated. If such a complete characterization is not carried out, the carbohydrate-specific protein may also be an enzyme of the cellular carbohydrate and glycoconjugate metabolism, see e.g. Roseman (Chem. Phys. Lipids 5 (1970), p. 270). In this publication of Roseman, the occurrence of glycosyl-transferases as cell surface-exposed carbohydrate-specific proteins has been suggested. This hypothesis was confirmed by e.g. Rauvala et al. (Proc. Natl. Acad. Sci. USA 80 (1983), p. 3991)

Finally, lectins were identified in chicken liver and embryonic chicken muscle (Ceri et al., J. Biol. Chem. 256 (1981, p. 390; de Waard et al., J.Biol.Chem. 252 (1976), p. 5781), human lung (J.T. Powell, Biochem. J. 187 (1980), p. 123) and human liver (Wild et al., Biochem. J. 210 (1983), p. 167).

Thus, lectins of mammalian tumor cells have not been characterized.

One object of the present invention therefore is the provision of specific carbohydrate-binding proteins (lectins) which are obtained from a mammalian tumor cell.

A further object of the present invention is the provision of specific carbohydrate-binding proteins (lectins) which are obtained from a mammalian tumor cell and which are responsible for specific surface properties of said tumor cell.

Still further, it is an object of this invention to provide a process for obtaining lectins from mammalian tumor cells which are responsible for specific surface properties.

In the process of the present invention the tumor tissue is first extracted with acetone, precipitating the protein and thus separating it from e.g. lipids. The acetone from the precipitate is then evaporated to obtain an acetone powder. The acetone powder is extracted with a suitable buffered aqueous solution in order to solubilize the lectin(s). Then the resulting aqueous extract is subjected to at least one affinity chromatography using columns to which carbohydrates which can be recognized by lectins are bound. Typical examples of such carbohydrates are lactose, asialofetuin, melibiose, mannan, fucose, invertase and heparin. Lactose and asialofetuin are classified as β-galactosides, melibiose as an α-galactoside. In this chromatography, lectins which are specifically recognizing the carbohydrate bound to the column will bind themselves to said columns. Subsequently these lectins are eluted from the column using an aqueous solution of the respective carbohydrate having e.g. a concentration of 0.3 or 0.5 M. Finally, the lectins are investigated with respect to their molecular weight and to their properties in hemagglutination-, enzyme activity- and aggregation-assays.

Essentially according to this method lectins of a rat rhabdomyosarcoma, a rat fibroadenoma, a rat invasive tubulopapillary adenocarcinoma with a low degree of differentiation, a rat non-invasive tubulopapillary adenocarcinoma with a high degree of differentiation, a murine teratoma, a human malignant epithelial tumor, a human teratocarcinoma (H12.1), a human embryonic carcinoma (H23), a human yolk sac carcinoma, a rat osteosarcoma and of a human sarcoma (Ewing's sarcoma) were isolated and characterized.

The lectins of the present invention can be used to provide corresponding monoclonal antibodies and subfragments thereof. Monoclonal antibodies, e.g. mouse or human antibodies, are isolated from suitable producer cells, e.g. hybridoma cell lines, according to known methods.

Anti-lectin-antibody-subfragments, such as the Fab and F(ab'2) fragments can be prepared by proteolytic cleavage of the antibody molecule with the enzymes papain and pepsin, respectively, followed by purification.

The monoclonal anti-lectin-antibodies or their subfragments, or lectins, or carbohydrates which are recognizable by said lectins can be conjugated with a chemotherapeutic or biologically active compound (such as 5-fluoruridine, vincristine, daunomycine or methothrexate), with a fluorescent or radioactively labelled group or with another compound permitting the detection of said molecules in a suitable assay for differential diagnosis of tumor types and the developmental stage of tumors.

Finally, the molecules referred to above can be used to provide diagnostic and pharmaceutical compositions, useful for rapid, reliable and precise clinical diagnosis, for scientific research, and for highly specific tumor therapy and inhibition of metastasation in mammalians and preferentially in humans. The pharmaceutical compositions containing at least one type of said carbohydrates are applicable also in a

4

state of neoplastic disease, where the risk of metastasation is strongly increased, e.g. after surgical treatment.

The molecules referred to above can be utilized in a composition such as tablet, capsule, solution or suspension. They may be compounded in conventional manner with a physiologically acceptable vehicle or carrier, excipient, binder, preservative, stabilizer, flavor, etc. as called for by accepted pharmaceutical practice.

In a first embodiment of the present invention five tumor types were investigated biochemically for the presence and characteristics of endogenous lectins.

A rat rhabdomyosarcoma reveals only $Ca^{2+}$-independent-lectin-specificities.

A rat fibroadenoma of the mammary gland was also investigated. It contains a diverse pattern of lectins. The lectin pattern of a spontenaous invasive rat tubulopapillary adenocarcinoma of the mammary gland is also diverse.

Additionally, a spontaneously occurring non-invasive rat tubulopapillary adenocarcinoma of the mammary gland was analyzed.

The tubulopapillary adenocarcinomas differ in their degree of differentiation and malignancy; the first one has a lower degree of differentiation. Since fibroadenoma and tubulopapillary adenocarcinoma of the rat mammary gland are morphologically similar to their counterparts in humans, these studies on the pattern of carbohydrate-binding lectins also have significance for human breast cancer.

Extracts using 0.2 M NaCl (salt) and 2 % Triton®X-100 (detergent) from a murine teratoma contain at least nine different lectins.

Furthermore, according to the present invention the pattern of different endogenous lectins of a human malignant epithelial tumor was investigated.

Additionally, according to the present invention three human testicular tumors were analyzed, namely a human teratocarcinoma (H12.1), a human embryonic carcinoma (H23), and a human yolk sac carcinoma.

Furthermore, the lectin pattern of two sarcomas was analyzed, namely of a rat osteosarcoma and of a human sarcoma (Ewings's sarcoma).

The lectins of the present invention which were isolated from said tumors and which were not known from any normal mammalian tissue are summarized in Table I.

## Table I

Endogenous tumor-derived lectins of the present invention

| Tumor type | Ca²⁺-dependent | | | | | Ca²⁺-independent | | | | |
|---|---|---|---|---|---|---|---|---|---|---|
| | Lactose | Asialofetuin | Melibiose | Mannan | Fucose | Lactose | Asialofetuin | Melibiose | Mannan | Fucose |
| Rat rhabdomyosarcoma | – | – | – | – | – | – | – | 29 43 45 | 60–72 | 60–72 |
| Rat fibroadenoma | 52 67 130 | 52 67 130 | 52 67 74 | 52 67 | 29 67 | – | – | – | – | – |
| Rat invasive tubulopapillary adenocarcinoma (low degree of differentiation) | 32 64 | – | – | – | 140 | 22 52 | n.d. | n.d. | 44 46 | 13 30 42 45 62 |
| Rat non-invasive tubulopapillary adenocarcinoma (high degree of differentiation) | – | – | – | – | 29 35 | – | – | 29 50 52 | – | 29 31 50 52 |
| Murine teratoma | 24 | – | – | 32 | 32 | – | – | – | – | ~100 |
| Human malignant epithelial tumor | 70 | – | 28 43 45 | – | 62 70 | – | – | 64 | – | 62 |
| Human terato-carcinoma (H12.1) | – | – | 31 | – | 31 70 | – | – | – | 68 | – |
| Human embryonic carcinoma (H23) | – | – | 56 66 | 66 | 31 | 32 | – | – | – | – |
| Human yolk sac carcinoma | 56 | – | 56 | – | 29 56 | 29 56 | 29 56 | 29 56 | – | 29 56 |
| Rat osteosarcoma | 64 | – | – | – | – | – | – | – | – | – |
| Human sarcoma (Ewing's sarcoma) | 52 56 | 52 56 | 52 56 | – | – | – | – | – | – | – |

The apparent molecular weight is given in thousands; n.d. = not determined

From the above results demonstrating that tumor cells carry specific lectins on their surface, the present inventors concluded that lectins and carbohydrates recognizable by lectins which are located on the surface of tumor cells play a key role in the communication between tumor cells as well as between tumor and "normal" cells and furthermore in the process of tissue specific metastasation (homotypic and heterotypic aggregation, see Fig. 1A and B). This provides an experimental basis for lectin impact on growth control and proliferation. If neoplastic cells expose both the lectin and the corresponding carbohydrate and if

endogenous lectins can have growth stimulating effects like ConA or PHA, then the tumor cells could stimulate themselves autocatalytically to exponential growth via the lectin-carbohydrate system. This phenomenon may indeed be observed in tumor colonies in vitro and in vivo. Of course, only the two together, the glycoconjugate and its lectin make sense in biological function.

From the above conclusions of the inventors it can be taken that in contrast to conventional tumor markers, the endogenous lectins are functional tumor markers, which participate in processes of tumor growth and spread.

Therefore, the lectin pattern of a tumor in principle can be characteristic for

- the type of tumor as compared to the nontransformed cell type,
- the developmental stage or degree of differentiation of the particular tumor,
- the tissue environment of the particular tumor.

Thus, the results of the experiments of the present invention permit the conception of new compositions useful for diagnosis and therapy of said tumors and also of functional tests to detect tumor cells at early stages of malignancy.

The diagnostic compositions of the present invention are based on the principle that either the tumor cell specific lectin(s) is (are) detected by the corresponding carbohydrate(s) or by corresponding monoclonal antibodies or antibody-subfragments or the tumor cell specific carbohydrate(s) is (are) detected by the corresponding lectin(s). For the purpose of suitable assays either the lectin(s), the carbohydrate(s), the monoclonal antibodies or the antibody-subfragments are conjugated with a biologically active compound or a compound permitting the detection of the respective molecules in said assay or alternatively the carbohydrates are radioactively labelled. Examples of useful markers or labels are enzymes, fluorochromes or spin labels. It is obvious to a person skilled in the art that immunological assays as RIA (radioimmunoassay) or ELISA (enzyme-linked immunosorbent assay) may be carried out by using the respective monoclonal anti-lectin-antibodies or their subfragments.

Advantageously the diagnostic compositions of this invention permit a rapid, reliable, and precise analysis of tumor cells and are therefore inter alia useful for the determination of malignancy of tumors during surgical treatments, for the differential diagnosis for the distinction of tumor types, and for the determination of the developmental stage of a tumor.

As to the pharmaceutical compositions, as yet the difficult objective in the chemotherapy of cancer and certain other diseases was the lacking selectiveness of the applicated drugs.

A main feature of the pharmaceutical compositions of the present invention is the highly specific interaction of lectins and carbohydrates on the one hand and of lectins and monoclonal antibodies or their subfragments on the other hand. For better understanding the revolutionary effectiveness of said therapeutic compositions some examples are given in the following.

If chemotherapeutic (e.g. methotrexate) or biologically active substances (e.g. a subunit of the cholera toxin) are conjugated with lectin(s) or with suitable carbohydrate(s) or with monoclonal anti-lectin-antibodies or subfragments thereof (i.e. synthesis of immunotoxins), they can be specifically targeted to tumor cells carrying the specific carbohydrates or the specific lectins. The specific action of the drug on tumor cells and only on these excludes essentially the side reactions of classical chemotherapeutic agents.

Furthermore the metastasation of tumors can be inhibited by oral or intravenous administration of suitable carbohydrates in such amounts that a complex or complexes with their corresponding lectin(s) are formed. Thus, they are blocking the binding site for adhesion between tumor cell and cells of the target organ.

The figures show:

Figure 1 shows a model for heterotypic and homotypic cell aggregation via the glycoconjugate-lectin system.

Figure 2 shows the inhibition of homotypic human teratocarcinoma cell aggregation by sugars. The aggregation in the absence of the inhibitor is given in (a), the aggregation in the presence of varying concentrations of L-fucose (o), D-galactose (x) and D-mannose (+) after 15 minutes is given in (b).

The following examples further illustrate the invention.

Example 1

Isolation and characterization of lectins derived from mammalian tumor cells

The pattern under investigation includes specificities for $\alpha$- and $\beta$-galactosides, $\alpha$-mannosyl and $\alpha$-fucosyl residues. It is divided into categories for dependence of the binding activity on the presence of $Ca^{2+}$

and on the extraction conditions, representing soluble intra- and extracellular proteins and integral membrane proteins.

Typically, aceton powder from frozen and thawed tumor tissue (50 g) was prepared by two successive extractions with 6 volumes of -70°C acetone in a Waring Blendor. The resulting 21 g of powder were extracted twice with 120 ml Buffer A (0.02 M Tris/HCl, pH 7.8, containing 0.2 M NaCl, 1 mM dithiothreitol and 0.01 mM phenylmethanesulfonyl-fluoride). The supernatants were combined and brought to a final concentration of 0.5 % Triton X-100®, 25 mM $CaCl_2$ and 1.25 M NaCl. The residual pellet was extracted twice with 120 ml of Buffer B (0.02 M Tris/HCl, pH 7.8, containing 0.4 M KCl, 2 % Triton X-100®, 1 mM dithiothreitol and 0.01 mM phenylmethanesulfonyl fluoride), the extracts were combined and adjusted to a concentration of 25 mM $CaCl_2$. Both Solutions were separately passed over a set of five columns (0.9 x 11 cm lactose-, asialofetuin-, melibiose- and mannan-Sepharose 4B®, 0.5 x 10 cm fucose-Sepharose 4B®, equilibrated with Buffer C (0.02 M Tris/HCl, pH 7.8, containing 1.25 M NaCl, 25 mM $CaCl_2$, 0.05 % Triton X-100® and 1 mM dithiothreitol). The column resins (lactose-, asialofetuin-, melibiose-, mannose- and fucose-Sepharose 4B®, using Sepharose 4B® from Pharmacia, Freiburg, FRG and Carbohydrates from Sigma, Munich, FRG) have been prepared after suitable activation (dinvinyl sulphone, cyanogen bromide from Merck, Darmstadt, FRG) according to standard procedures. Also reductive amination of disaccharides to an amino ethylated polyacrylamide support is possible. Asialofetuin was prepared from fetuin (Sigma, Munich, FRG) by desialylation at pH 2 and 80°C. After extensive washing of the columns, elution of the $Ca^{2\oplus}$-dependent carbohydrate-binding proteins from the salt extract and the detergent extract was performed using Buffer D (Buffer C containing 4 mM EDTA instead of 25 mM $CaCl_2$).

The material was pooled, readjusted to 25 mM $CaCl_2$ and adsorbed to a smaller column of the corresponding resin (3 ml volume). As was noted before, it is advantageous to perform the second elution with the specific sugars (0.5 M lactose, 0.5 M melibiose, 0.5 M D-mannose, 0.5 M L-fucose). In general, elution with a molarity of 0.3 has proved sufficient for complete elution of lectins. The $Ca^{2\oplus}$-independent carbohydrate-binding proteins were eluted by application of Buffer C + 0.5 M of the specific sugar from the first set of columns that had been reequilibrated with Buffer C. The sugar was removed by dialysis and affinity chromatography was repeated on small columns (3 ml).

Specificity at this stage was checked by unsuccessful binding of lectins in the presence of specific sugars (0.3M) and by in vitro binding to cytochemical markers on nitrocellulose (Gabius et al., J. Natl. Cancer Inst. 73 (1984), p. 1349). Furthermore, bound lectins were not elutable by unspecific sugars like sucrose.

This procedure allowed the separation of $Ca^{2+}$-dependent lectins from $Ca^{2+}$-independent lectins. In this example five carbohydrate specificities were tested. Of course, many other specificities can be tested in the same way, such as specificities for neuraminic acid, rhamnose, heparin, galactosamine, glucosamine and methylated and acetylated derivatives of these carbohydrates.

The samples were concentrated by ultrafiltration using a membrane as filter (YM5 filter, Amicon). All lectins were characterized with respect to homogeneity and molecular weight by polyacrylamide gel electrophoresis in the presence of 0.1 % sodium dodecyl sulfate on 20 x 20 cm 10 % running gels with a 3 % stacking gel. The gels were stained with Coomassie blue for heparin-inhibitable lectin or by the silver staining method according to Morrissey (Anal. Biochem. 117 (1981), p. 307) for all other samples.

Standards for molecular weight designation were: phosphorylase b (97 kDa), bovine serum albumin (66 kDa), egg albumin (44 kDa), glyceraldehyde-3-phosphate dehydrogenase (36 kDa), carbonic anhydrase (29 kDa), trypsinogen (24 kDa), β-lactoglobin (18.4 kDa).

Furthermore the lectins were optionally characterized by hemagglutination, enzyme and aggregation assays. Lectin activity was assayed in microtiter plates with V-shaped bottoms with glutaraldehyde-fixed, trypsin-treated rabbit erythrocytes that in the case of heparin-inhibitable lectin were pretreated with ethanol. All agglutination assays were scored after 1 h at room temperature.

Enzyme assays for β-galactosidase, sialyltransferase and fucosyltransferase, using asialofetuin as potential acceptor, α-mannosidase and α-fucosidase were performed. The sensitivity of the assay varies from a detection limit of $1.5 \times 10^{-8}$ unit of enzyme activity for transferases to $5 \times 10^{-4}$ unit of enzyme for glycosidases (1 unit = μmol of substrate converted per min). Aggregation of cerebroside vesicles (12 mol % N-plamitoyl-DL-dihydrolactocerebroside) by β-galactoside specific lectins was performed at a concentration of 7 μg/ml lectin.

With each isolation procedure identical results were achieved at least twice.

Example 2

Isolation and characterization of lectins derived from a spontaneous rat rhabdomyosarcoma, a rat fibroadenoma, two rat tubulopapillary adenocarcinomas and a murine teratoma

Basically according to the procedure described in Example 1 five different tumor types were investigated biochemically for the presence and characteristics of endogenous carbohydrate-binding proteins (lectins).

The tumors had developed spontaneously in female rats or mice which were obtained from the breeding colonies of the Central Institute for Laboratory Animal Breeding, Lettow-Vorbeck-Allee 57, 3000 Hannover 91, West Germany.

The rat rhabdomyosarcoma originated from the thoracic cavity of an inbred Brown Norway rat (BN/Han) attached to the cranioventral section of the sternum and the ribs. The second tumor, a fibroadenoma of the mammary gland, was found in a 9 months old femal breeder rat of the Han: SPRD outbred stock which was removed from the breeding colony of the institute for routine hygienic monitoring. The third and the fourth tumor can both be classified as belonging to the tubulopapillary adenocarcinoma group. The third tumor, an invasive tubulopapillary adenocarcinoma, had developed in the inguinal area of an outbred Sprague-Dawley rat (Han:SPRD) and was observed in a life-span study maintaining rats from weaning up to their natural deaths. The fourth tumor, an non-invasive rat tubulopapillary adenocarcinoma displays a higher degree of differentiation than the third tumor and was obtained from an inbred BDII/Han rat.

The murine teratoma developed in a 6 months old Han:NMRI mouse in the left ovary. This mouse teratoma was well differentiated, consisting of various tissues as bone, cartilage, connective tissue, striated and smooth muscle cells, nervous tissue inlcuding retina, and an epithelial component.

The purification and characterization of lectins started from 6 g of a rat rhabdomyosarcoma, 22.7 g of a rat fibroadenoma, 27 g of an invasive rat tubulopapillary adenocarcinoma, 15.5 g of a non-invasive tubulopapillary adenocarcinoma and 28 g of a murine teratoma.

The rat rhabdomyosarcoma was homogenized in 6 volumes of extraction medium (75 mM $Na_2HPO_4/KH_2PO_4$, pH 7.2, 75 mM NaCl, 4 mM $\beta$-mercaptoethanol, 2 mM EDTA and 0.01 mM phenylmethanesulfonyl fluoride (MEPBS) containing 1 M NaCl, 0.2 M lactose and 0.2 M mannose). After centrifugation and dialysis first against MEPBS, later against a buffer with Tris-HCl (75 mM) instead of phosphate, raised successively from pH 7.5 to pH 7.8, the solution was adjusted to 20 mM $CaCl_2$ and sucessively passed over a set of columns (o.9 x 12 cm) equilibrated with buffer A (75 mM Tris-HCl, pH 7.8, 25 mM $CaCl_2$, 4 mM $\beta$-mercaptoethanol, 2 mM EDTA, 0.01 mM phenylmethanesulfonyl fluoride and 1 M NaCl). The columns were processed as in Example 1. The extract, after passing over the columns, was concentrated, submitted to a column chromatography with Sepharose CL-2B® (Pharmacia, Freiburg, FRG) and dialyzed in the presence of 40 ml heparin-Sepharose 4B® against 0,01 M Tris-HCl, pH 8.6, 4mM $\beta$-mercaptoethanol and 0.3 M NaCl. Elution from the columns was performed by two means:

a) with buffer A after omission of $CaCl_2$ and addition of 4 mM EDTA,

b) after reequilibration with buffer A using buffer A + 0.5 M of the appropriate sugar (lactose, melibiose, mannose, fucose).

After dialysis of the samples against buffer A, the affinity chromatography for analysing the lectin pattern was repeated using columns with a capacity of 5 ml.

The lectin pattern of the other tumors was analysed exactly as described in Example 1.

Furthermore, the lectins isolated from said rat and mouse tumors were subjected to characterization by gel electrophoresis, hemagglutination assays and enzymatic assays as described in Example 1.

The lectins which were obtained and which were not known from any type of normal mammalian tissue are given in Table I.

Example 3

Isolation and characterization of lectins derived from a human malignant epithelial tumor

The tumor was surgically removed from the left flexura of the colon of a 60 year old woman.

The preparation of the lectins from the tumor tissue was carried out as described in Example 1 starting with 34 g frozen and thawed tumor material.

The preparation was carried out three times with identical results.

Subsequently the lectins of this epithelial tumor were further characterized by demonstrating hemagglutinating activity and excluding any detectable enzymatic activity according to the methods given in Example 1.

The lectins obtained from this human epithelial tumor which were not known from any type of normal mammalian tissue are summarized in Table I.

Example 4

Isolation and characterization of lectins derived from different human testicular tumors

Essentially according to the procedure given in Example 1 the lectin pattern of a human teratocarcinoma was analyzed.

The teratocarcinoma cell line H12.1 had been established from a primary human testis tumor and was subcultured more than 60 times in vitro. The culture was maintained in RPMI 1640 medium (Flow Labs, Meckenheim, FRG) containing 15 % heat inactivated fetal calf serum (Biochrom, Berlin, FRG), 10 % tryptose phosphate broth (Flow Labs, Meckenheim, FRG), 2 mM L-glutamin, 100 I.U./ml penicillin and 100 μg/ml streptomycin. For transplantation athymic nude NMRI mice (nu/nu, 6 - 8 weeks old) were treated subcutaneously with approximately $10^7$ cells. After 2 months the tumors were removed, immediately frozen in liquid nitrogen and stored at -80° C. For the preparation of lectin from the culture, cells were washed with buffer (75 mM Tris/HCl, pH 7.8, containing 1 mM phenylmethanesulfonyl fluoride, 2 mM dithiothreitol and 1 mM $NaN_3$), scraped out and frozen. Homogenization of 1 g cells (wet weight) was carried out with the same buffer containing 2 % Triton X-100® and lacking $NaN_3$.

In a typical preparation of lectins, acetone powder of tumor material (14 g) was extracted and fractionated as given in Example 1.

All samples after two cycles of affinity chromatography were concentrated by ultrafiltration using a membrane as filter (Diaflo Ultrafiltration Model 50 with a YM-5 membrane). Detergent was removed by chloroform extraction and the heparin-specific lectin was isolated from the tumor material. Subsequently, the lectin pattern was analysed according to example 1, including tests for hemagglutinating and enzyme activity.

To demonstrate the possible functional role of the lectins of said human teratocarcinoma cells, the binding of erythrocytes to these teratocarcinoma cells was monitored in a simple visual assay using trypsinized, glutaraldehyd-fixed rabbit erythrocytes (rosette formation). Since carbohydrate structures on the surface of erythrocytes apparently are recognized by carbohydrate-binding proteins of the teratocarcinoma cells during the heterotypic recognition, inhibition of this process by addition of sugars and glycoproteins was tested (Table II).

## Table II
### Inhibition of rosette formation

| Inhibitor | % Inhibition of rosette formation |
|---|---|
| N-acetyl-D-galactosamine | 0 |
| L-fucose | 2 |
| D-galactose | 4 |
| N-acetyl-D-glucosamine | 0 |
| D-mannose | 10 |
| fetuin | 0 |
| asialofetuin | 7 |
| asialo-agalactofetuin | 0 |
| mannan | 21 |
| Invertase | 34 |
| Invertase (periodate-oxidized) | 4 |
| lactose-BSA | 7 |
| mannose-BSA | 14 |

Saccharides were added at 0.2 M, glycoproteins at 1 mg/ml. All results are averages from 8 - 10 independent experiments.

10

Whereas monosaccharids as D-mannose and D-galactose only slightly inhibited the heterotypic aggregation at 0.2 M concentration, a more pronounced effect was seen with glycosylated bovine serum albumin (lac-BSA, man-BSA). Since galactose-binding proteins were known to bind to the mannose-glycoprotein invertase, the difference in inhibitory efficiency of invertase in relation to mannan may indicate a binding of invertase to galactose- and mannose-specific sites on the teratocarcinoma cells. No inhibition was seen with N-acetylglucosamine, asialoagalactofetuin, fetuin, glucose and sucrose. This excludes an unspecific sugar effect on rosette formation. Bovine serum albumin (BSA) also had no inhibitory influence. Coupling of p-aminophenyl-glucoside by diazotation to BSA, as similarly used for the derivatives of $\beta$-lactose and $\alpha$-D-mannose, does not influence the inertness of BSA in rosette formation, excluding any uspecific effect due to the chemical modification procedure. Since the inhibition by invertase is drastically reduced after extensive oxidation of sugar moieties in invertase by periodate treatment, the importance of sugars in the recognitive process during rosette formation is further emphasized.

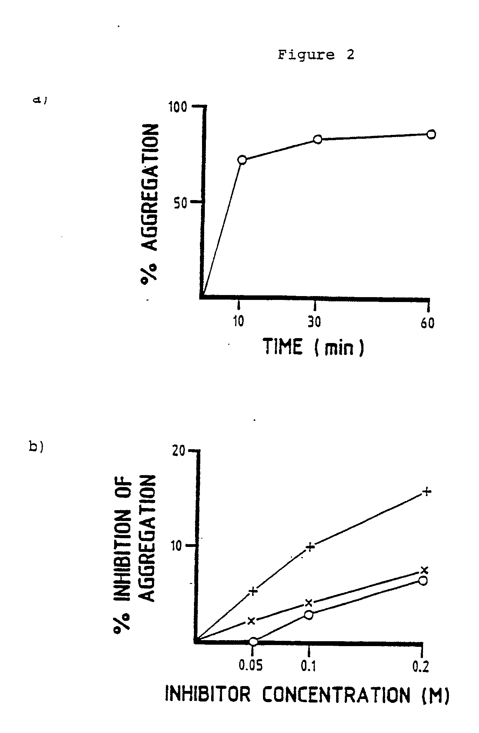

Inhibition of rosette formation carried out as described above demonstrated the participation of a protein carbohydrate interaction in heterotypic aggregation. Since type and abundancy of carbohydrate structures of glycoconjugates on erythrocytes and teratocarcinoma cells might differ significantly, the influence of sugars on reaggregation of teratocarcinoma cells that were carefully mechanically dissociated in calcium- and magnesiumfree phosphate-buffered saline was tested. The tests revealed a similar inhibition pattern for the homotypic aggregation in relation to the heterotypic system with mannose and galactose being effective inhibitors (Fig. 2). The ability of D-mannose and D-galactose to inhibit aggregation of teratocarcinoma cells further suggested that these sugars interact with cell surface carbohydrate-binding proteins. In order to establish the capacity of sugars to bind to carbohydrate-binding proteins of the cell surface, fluorescent, glycosylated markers provided a versatile cytological tool for visualization. Observation of teratocarcinoma cells after labelling with markers specific for D-mannose and lactose resulted in significant binding of markers. The binding was performed at 4°C in order to minimize internalization or shedding of membrane-bound proteins. Complete inhibition of binding occured in the presence of the appropriate sugar (D-mannose or lactose) in a 0.25M concentration or of 1 mg/ml unlabelled glycosylated BSA. These data raised evidence that the lectins contained in the detergent extract of human teratocarcinoma cells being specific for D-mannose and D-galactose are involved in $Ca^{2+}$-independent cell-cell recognition.

The human embryonic carcinoma cell line H 23 had been established from a primary human testicular tumor of a 26 year old patient and was subcultured more than 30 times in vitro. For transplantation, athymic nude mice (nu/nu, 6 - 8 weeks old) (Central Institute for Animal Breeding, supra) were injected subcutaneously with approximately $10^7$ cells. After two months the tumors, histologically determined as embryonic carcinoma, were removed, immediately frozen in liquid nitrogen and stored at -80°C.

The yolk sac tumor material was obtained by autopsy of a 20 year old boy suffering from a testicular embryonic carcinoma. Viable parts of the abdominal tumor mass were removed, consisting histologically predominantly of yolk sac tumor material.

The lectins of said human embryonic carcinoma cell line H 23 and of said yolk sac tumor were isolated and characterized according to Example 1, including functional tests for hemagglutinating and enzyme activity.

The lectins identified in the above-mentioned human testicular tumors which were not known from any type of normal mammlian tissue are summarized in Table I.

The analyzed lectin patterns demonstrate that lectins can be considered as functional tumor markers useful for differential diagnosis of tumors and different developmental stages of tumors.

## Example 5

Isolation and characterization of lectins derived from a rat osteosarcoma and a human sarcoma (Ewing's sarcoma)

The lectin pattern of a rat osteosarcoma was determined by analysis of 3 g tumor tissue as described in Example 1.

Tumor material of the human sarcoma (Ewing's sarcoma) was obtained from the Cancer Center of the University of California, San Diego. 3.5 g of the tissue were extracted and analyzed for their lectin pattern as described in Example 1.

Subsequently, functional tests for hemagglutinating and enzyme activity were carried out according to Example 1.

The lectins obtained from these tumors which were not known from any type of normal mammalian tissue are summarized in Table I.

Claims

Claims for the following Contracting States: BE, CH, DE, FR, GB, IT, LI, LU, NL, SE

1. Carbohydrate-binding proteins (lectins) obtained from a mammalian tumor cell, which specifically occur on the surface of tumor cells but not on the surface of normal cells.

2. Lectins according to claim 1, characterized in that they are derived from a rat rhabdomyosarcoma, have the following molecular weight (in thousands) and bind specifically to the following carbohydrates:

$Ca^{2+}$-independent

| Lactose | Asialofetuin | Melibiose | Mannan | Fucose |
|---------|--------------|-----------|--------|--------|
| – | – | 29 | 60-72 | 60-72 |
|   |   | 43 |   |   |
|   |   | 45 |   |   |

3. Lectins according to claim 1, characterized in that they are derived from a rat invasive tubulopapillary adenocarcinoma with a low degree of differentiation, have the following molecular weight (in thousands) and bind specifically to the following carbohydrates:

$Ca^{2+}$-dependent

| Lactose | Asialofetuin | Melibiose | Mannan | Fucose |
|---------|--------------|-----------|--------|--------|
| 32 |   |   |   |   |
| 64 | – | – | – | 140 |

$Ca^{2+}$-independent

| Lactose | Asialofetuin | Melibiose | Mannan | Fucose |
|---------|--------------|-----------|--------|--------|
| 22 | – | – | 44 | 13 |
| 52 |   |   | 46 | 30 |
|   |   |   |   | 42 |
|   |   |   |   | 45 |
|   |   |   |   | 62 |

4. Lectins according to claim 1, characterized in that they are derived from a murine teratoma, have the following molecular weight (in thousands) and bind specifically to the following carbohydrates:

$Ca^{2+}$-dependent

| Lactose | Asialofetuin | Melibiose | Mannan | Fucose |
|---------|--------------|-----------|--------|--------|
| 24 | – | – | 32 | 32 |

$Ca^{2+}$-independent

| Lactose | Asialofetuin | Melibiose | Mannan | Fucose |
|---------|--------------|-----------|--------|--------|
| – | – | – | – | ≈100 |

5. Lectins according to claim 1, characterized in that they are derived from a human malignant epithelial tumor, have the following molecular weight (in thousands) and bind specifically to the following carbohydrates:

$Ca^{2+}$-dependent

| Lactose | Asialofetuin | Melibiose | Mannan | Fucose |
|---------|--------------|-----------|--------|--------|
| 70 | - | 28 | - | 62 |
| | | 43 | | 70 |
| | | 45 | | |

$Ca^{2+}$-independent

| Lactose | Asialofetuin | Melibiose | Mannan | Fucose |
|---------|--------------|-----------|--------|--------|
| - | - | 64 | - | 62 |

6. Lectins according to claim 1, characterized in that they are derived from a human teratocarcinoma, have the following molecular weight (in thousands) and bind specifically to the following carbohydrates:

$Ca^{2+}$-dependent

| Lactose | Asialofetuin | Melibiose | Mannan | Fucose |
|---------|--------------|-----------|--------|--------|
| - | - | 31 | - | 31 |
| | | | | 70 |

$Ca^{2+}$-independent

| Lactose | Asialofetuin | Melibiose | Mannan | Fucose |
|---------|--------------|-----------|--------|--------|
| - | - | - | 68 | - |

7. Process for obtaining the lectins according to claims 1 to 6 comprising the following steps:
   - extraction of the tumor tissue with acetone;
   - evaporation of the acetone extract to obtain an acetone powder;
   - extraction of the acetone powder with a buffered aqueous solution for the solubilization of the lectins;
   - adsorption of the lectins contained in the aqueous extract to affinity chromatography columns to which carbohydrates which can be recognized by lectins are bound;
   - elution of $Ca^{2+}$-dependent lectins from the column with an aqueous solution of a chelating agent; and subsequently
   - elution of $Ca^{2+}$-independent lectins from the column with an aqueous solution of a carbohydrate competing with the carbohydrate bound to the column for the binding site of the lectin.

8. Process according to claim 7, characterized in that the aqueous extract is obtained by homogenisation of the tumor tissue in an aqueous extraction medium and by centrifugation and dialysis of the homogenisate.

9. Monoclonal antibody or a subfragment thereof derived from a human or a murine hybridoma, characterized in that it is specifically directed to a lectin according to any one of claims 1 to 6.

10. Diagnostic composition for the detection of mammalian tumor cells, characterized in that it contains a lectin according to any one of claims 1 to 6, a monoclonal antibody or a subfragment thereof which is specifically directed to a lectin according to any one of claims 1 to 6 or a derivative of said lectin or said monoclonal antibody.

11. Use of a lectin according to any one of claims 1 to 6, a monoclonal antibody or a subfragment thereof which is specifically directed to a lectin according to any one of claims 1 to 6, or of a derivative of said lectin or antibody for the production of a diagnostic composition for the detection of mammalian tumor cells.

12. Use of a carbohydrate recognizable by a lectin according to any one of claims 1 to 6 or of a derivative of said carbohydrate for the production of a diagnostic composition for the detection of mammalian tumor cells.

13. Pharmaceutical composition for treating malignant neoplasias by specifically destroying tumor cells and/or inhibiting metastasation containing an effective amount of a lectin according to any one of claims 1 to 6, a monoclonal antibody or a subfragment thereof which is specifically directed to a lectin according to any one of claims 1 to 6 or a derivative of said lectin, or said monoclonal antibody and a pharmaceutically acceptable carrier or diluent.

14. Use of a carbohydrate recognizable by a lectin according to any one of claims 1 to 6 or of a derivative of said carbohydrate for the production of a pharmaceutical composition for the treatment of malignant neoplasias by specifically destroying tumor cells and/or inhibiting metastasation.

**Claims for the following Contracting State: AT**

1. A process for obtaining carbohydrate-binding proteins (lectins) from a mammalian tumor cell, said lectins specifically occuring on the surface of tumor cells but not on the surface of normal cells, and said method comprising the following steps:
   - extraction of the tumor tissue with acetone;
   - evaporation of the acetone extract to obtain an acetone powder;
   - extraction of the acetone powder with a buffered aqueous solution for the solubilization of the lectins;
   - adsorption of the lectins contained in the aqueous extract to affinity chromatography columns to which carbohydrates which can be recognized by lectins are bound;
   - elution of $Ca^{2+}$-dependent lectins from the column with an aqueous solution of a chelating agent; and subsequently
   - elution of $Ca^{2+}$-independent lectins from the column with an aqueous solution of a carbohydrate competing with the carbohydrate bound to the column for the binding site of the lectin.

2. The process according to claim 1, characterized in that the aqueous extract is obtained by homogenisation of the tumor tissue in an aqueous extraction medium and by centrifugation and dialysis of the homogenisate.

3. The process according to claim 1 or 2, wherein the lectins are characterized in that they are derived from a rat rhabdomyosarcoma, have the following molecular weight (in thousands) and bind specifically to the following carbohydrates:

## $Ca^{2+}$-independent

| Lactose | Asialofetuin | Melibiose | Mannan | Fucose |
|---------|--------------|-----------|--------|--------|
| —       | —            | 29        | 60-72  | 60-72  |
|         |              | 43        |        |        |
|         |              | 45        |        |        |

4. The process according to claim 1 or 2, wherein the lectins are characterized in that they are derived from a rat invasive tubulopapillary adenocarcinoma with a low degree of differentiation, have the following molecular weight (in thousands) and bind specifically to the following carbohydrates:

14

Ca$^{2+}$-dependent

| Lactose | Asialofetuin | Melibiose | Mannan | Fucose |
|---|---|---|---|---|
| 32 | | | | |
| 64 | - | - | - | 140 |

Ca$^{2+}$-independent

| Lactose | Asialofetuin | Melibiose | Mannan | Fucose |
|---|---|---|---|---|
| 22 | - | - | 44 | 13 |
| 52 | | | 46 | 30 |
| | | | | 42 |
| | | | | 45 |
| | | | | 62 |

5. The process according to claim 1 or 2, wherein the lectins are characterized in that they are derived from a murine teratoma, have the following molecular weight (in thousands) and bind specifically to the following carbohydrates:

Ca$^{2+}$-dependent

| Lactose | Asialofetuin | Melibiose | Mannan | Fucose |
|---|---|---|---|---|
| 24 | - | - | 32 | 32 |

Ca$^{2+}$-independent

| Lactose | Asialofetuin | Melibiose | Mannan | Fucose |
|---|---|---|---|---|
| - | - | - | - | ≈100 |

6. The process according to claim 1 or 2, wherein the lectins are characterized in that they are derived from a human malignant epithelial tumor, have the following molecular weight (in thousands) and bind specifically to the following carbohydrates:

Ca$^{2+}$-dependent

| Lactose | Asialofetuin | Melibiose | Mannan | Fucose |
|---|---|---|---|---|
| 70 | - | 28 | - | 62 |
| | | 43 | | 70 |
| | | 45 | | |

Ca$^{2+}$-independent

| Lactose | Asialofetuin | Melibiose | Mannan | Fucose |
|---|---|---|---|---|
| - | - | 64 | - | 62 |

7. The process according to claim 1 or 2, wherein the lectins are characterized in that they are derived

15

from a human teratocarcinoma, have the following molecular weight (in thousands) and bind specifically to the following carbohydrates:

**Ca$^{2+}$-dependent**

| Lactose | Asialofetuin | Melibiose | Mannan | Fucose |
|---|---|---|---|---|
| - | - | 31 | - | 31 |
| | | | | 70 |

**Ca$^{2+}$-independent**

| Lactose | Asialofetuin | Melibiose | Mannan | Fucose |
|---|---|---|---|---|
| - | - | - | 68 | - |

8. A process for the production of a monoclonal antibody or a subfragment thereof specifically directed to a lectin as obtainable according to a process of any one of claims 1 to 7, said process comprising the isolation of said antibody from a human or a murine hybridoma and optionally proteolytic cleavage of the isolated antibody.

9. A process for the production of a diagnostic composition for the detection of mammalian tumor cells, characterized in that a lectin obtainable according to a process of any one of claims 1 to 7, a monoclonal antibody or a subfragment thereof obtainable according to the process of claim 8 or a derivative of said lectin or said monoclonal antibody or subfragment thereof is conjugated with a compound permitting its detection in a suitable assay for differential diagnosis of tumor types and the developmental stage of tumors.

10. Use of a lectin obtainable according to a process of any one of claims 1 to 7, a monoclonal antibody or a subfragment thereof obtainable according to the process of claim 8, or of a derivative of said lectin or antibody for the production of a diagnostic composition for the detection of mammalian tumor cells.

11. Use of a carbohydrate recognizable by a lectin obtainable according to a process of any one of claims 1 to 7 or of a derivative of said carbohydrate for the production of a diagnostic composition for the detection of mammalian tumor cells.

12. A process for the production of a pharmaceutical composition for treating malignant neoplasias by specifically destroying tumor cells and/or inhibiting metastasation comprising compounding an effective amount of a lectin obtainable according to a process of any one of claims 1 to 7, a monoclonal antibody or a subfragment thereof obtainable according to the process of claim 8, or of a derivative of said lectin or antibody with a pharmaceutically acceptable carrier or diluent.

13. Use of a carbohydrate recognizable by a lectin obtainable according to a process of any one of claims 1 to 7, or of a derivative of said carbohydrate for the production of a pharmaceutical composition for the treatment of malignant neoplasias by specifically destroying tumor cells and/or inhibiting metastasation.

**Revendications**

**Revendications pour les Etats contractants suivants: BE, CH, DE, FR, GB, IT, LI, LU, NL, SE**

1. Protéines de liaison des hydrates de carbone (lectines) qu'on obtient à partir d'une cellule tumorale de mammifères, qui apparaissent spécifiquement à la surface des cellules tumorales, mais non à la surface des cellules normales.

2. Lectines selon la revendication 1, caractérisées en ce qu'elles proviennent d'un rhabdomyosarcome de rat, présentent la masse moléculaire suivante (en milliers) et se lient spécifiquement aux hydrates de carbone suivants:

**Indépendantes de $Ca^{2+}$**

| Lactose | Asialofetuine | Melibiose | | Mannane | Fucose |
|---------|---------------|-----------|---|---------|--------|
| – | – | 29 | 60-72 | 60-72 | |
| | | 43 | | | |
| | | 45 | | | |

3. Lectines selon la revendication 1, caractérisées en ce qu'elles proviennent d'un adénocarcinome tubulopapillaire invasif du rat ayant un faible degré de différenciation, présentent les masses moléculaires suivantes (en milliers) et se lient spécifiquement aux hydrates de carbone suivants:

**Dépendantes de $Ca^{2+}$**

| Lactose | Asialofetuine | Melibiose | Mannane | Fucose |
|---------|---------------|-----------|---------|--------|
| 32 | | | | |
| 64 | – | – | – | 140 |

**Indépendantes de $Ca^{2+}$**

| Lactose | Asialofetuine | Melibiose | Mannane | Fucose |
|---------|---------------|-----------|---------|--------|
| 22 | – | – | 44 | 13 |
| 52 | | | 46 | 30 |
| | | | | 42 |
| | | | | 45 |
| | | | | 62 |

4. Lectines selon la revendication 1, caractérisées en ce qu'elles proviennent de teratome murin, possèdent la masse moléculaire suivante (en milliers) et se lient spécifiquement aux hydrates de carbone suivants:

**Dépendantes de $Ca^{2+}$**

| Lactose | Asialofetuine | Melibiose | Mannane | Fucose |
|---------|---------------|-----------|---------|--------|
| 24 | – | – | 32 | 32 |

**Indépendantes de $Ca^{2+}$**

| Lactose | Asialofetuine | Melibiose | Mannane | Fucose |
|---------|---------------|-----------|---------|--------|
| – | – | – | – | $\approx 100$ |

5. Lectines selon la revendication 1, caractérisées en ce qu'elles proviennent d'une tumeur épithéliale maligne humaine, ont la masse moléculaire suivante en milliers et se lient spécifiquement aux hydrates de carbone ci-après:

### Dépendantes de Ca$^{2+}$

| Lactose | Asialofetuine | Melibiose | Mannane | Fucose |
|---------|---------------|-----------|---------|--------|
| 70 | - | 28 | - | 62 |
| | | 43 | | 70 |
| | | 45 | | |

### Indépendantes de Ca$^{2+}$

| Lactose | Asialofetuine | Melibiose | Mannane | Fucose |
|---------|---------------|-----------|---------|--------|
| - | - | 64 | - | 62 |

6. Lectines selon la revendication 1, caractérisées en ce qu'elles proviennent d'un teratocarcinome humain, ont une masse moléculaire ci-après (en milliers) et se lient spécifiquement aux hydrates de carbone suivants:

### Dépendantes de Ca$^{2+}$

| Lactose | Asialofetuine | Melibiose | Mannane | Fucose |
|---------|---------------|-----------|---------|--------|
| - | - | 31 | - | 31 |
| | | | | 70 |

### Indépendantes de Ca$^{2+}$

| Lactose | Asialofetuine | Melibiose | Mannane | Fucose |
|---------|---------------|-----------|---------|--------|
| - | - | - | 68 | - |

7. Procédé pour obtenir les lectines selon les revendications 1 à 6, comportant les stades suivants:
   - extraction des tissus tumoraux avec de l'acétone ;
   - évaporation de l'extrait acétonique pour obtenir une poudre d'acétone ;
   - extraction de la poudre d'acétone avec une solution aqueuse tamponnée pour la solubilisation des lectines ;
   - adsorption des lectines contenues dans l'extrait aqueux sur les colonnes de chromatographie par affinité auxquelles sont liés les hydrates de carbone qui peuvent être reconnus par les lectines ;
   - élution des lectines dépendantes de Ca$^{2+}$ à partir de la colonne à l'aide d'un agent chélateur en solution aqueuse ; et, ensuite,
   - élution de lectines indépendantes de Ca$^{2+}$ de la colonne à l'aide d'une solution aqueuse d'hydrate de carbone en compétition avec l'hydrate de carbone lié à la colonne pour le site de liaison de la lectine.

8. Procédé selon la revendication 7, caractérisé en ce qu'on obtient l'extrait aqueux par homogénéisation de tissus tumoraux dans un milieu aqueux d'extraction et par centrifugation et dialyse de l'homogénéisat.

9. Anticorps monoclonal ou un sous-fragment de celui-ci dérivé d'un hybridome humain ou murin, caractérisé en ce qu'il est spécifiquement dirigé vers une lectine selon l'une quelconque des revendications 1 à 6.

10. Composition de diagnostic pour la détection de tumeurs des mammifères, caractérisée en ce qu'elle contient une lectine selon l'une quelconque des revendications 1 à 6, un anticorps monoclonal ou un sous-fragment de celui-ci dirigé spécifiquement vers une lectine selon l'une quelconque des revendications 1 à 6, ou un dérivé de ladite lectine ou dudit anticorps monoclonal.

11. Utilisation d'une lectine selon l'une quelconque des revendications 1 à 6, d'un anticorps monoclonal ou d'un sous-fragment de celui-ci, qui est spécifiquement dirigé vers une lectine selon l'une quelconque des revendications 1 à 6 ou d'un dérivé de ladite lectine ou de l'anticorps pour la production d'une composition de diagnostic pour la détection des cellules tumorales chez les mammifères.

12. Utilisation d'un hydrate de carbone reconnaissable par une lectine selon l'une quelconque des revendications 1 à 6 ou d'un dérivé dudit hydrate de carbone pour la production d'une composition de diagnostic servant à la détection des cellules tumorales de mammifères.

13. Composition pharmaceutique pour le traitement de néoplasies malignes par destruction spécifique des cellules tumorales et/ou inhibition de la formation des métastases contenant une quantité efficace d'une lectine selon l'une quelconque des revendications 1 à 6, un anticorps monoclonal ou un sous-fragment de celui-ci spécifiquement dirigé vers une lectine selon l'une quelconque des revendications 1 à 6, ou un dérivé de ladite lectine, ou ledit anticorps monoclonal et un véhicule ou diluant pharmaceutiquement acceptable.

14. Utilisation d'un hydrate de carbone reconnaissable par une lectine selon l'une quelconque des revendications 1 à 6, ou d'un dérivé dudit hydrate de carbone pour la production d'une composition pharmaceutique destinée au traitement des néoplasies malignes par destruction spécifique des cellules tumorales et/ou inhibition de la formation des métastases.

**Revendications pour l'Etat contractant suivant: AT**

1. Procédé de production de protéines (lectines) de liaisons d'hydrates de carbone à partir d'une cellule tumorale de mammifères, lesdites lectines apparaissant spécifiquement à la surface des cellules tumorales, mais non à la surface des cellules normales et ledit procédé comprenant les étapes suivantes :
   - extraction des tissus tumoraux avec de l'acétone ;
   - évaporation de l'extrait acétonique pour obtenir une poudre d'acétone ;
   - extraction de la poudre d'acétone avec une solution aqueuse tamponnée pour la solubilisation des lectines ;
   - adsorption des lectines contenues dans l'extrait aqueux sur les colonnes de chromatographie par affinité auxquelles sont liés les hydrates de carbone qui peuvent être reconnus par les lectines ;
   - élution des lectines dépendantes de $Ca^{2+}$ à partir de la colonne à l'aide d'un agent chélateur en solution aqueuse ; et, ensuite,
   - élution de lectines indépendantes de $Ca^{2+}$ de la colonne à l'aide d'une solution aqueuse d'hydrate de carbone en compétition avec l'hydrate de carbone lié à la colonne pour le site de liaison de la lectine.

2. Procédé selon la revendication 1, caractérisé en ce qu'on obtient l'extrait aqueux par homogénéisation de tissus tumoraux dans un milieu aqueux d'extraction et par centrifugation et dialyse de l'homogénéisat.

3. Procédé selon la revendication 1 ou 2, dans lequel les lectines sont caracérisées en ce qu'elles proviennent d'un rhabdomyosarcome de rat, présentent la masse moléculaire suivante (en milliers) et se lient spécifiquement aux hydrates de carbone suivants:

Indépendantes de $Ca^{2+}$

| Lactose | Asialofetuine | Melibiose | Mannane | Fucose |
|---------|---------------|-----------|---------|--------|
| − | − | 29 | 60−72 | 60−72 |
| | | 43 | | |
| | | 45 | | |

4. Procédé selon la revendication 1 ou 2, dans lequel les lectines sont caractérisées en ce qu'elles proviennent d'un adénocarcinome tubulopapillaire invasif au rat, ayant un faible degré de différenciation, possèdent la masse moléculaire suivante (en milliers) et se lient spécifiquement aux hydrates de carbone suivants :

### Dépendantes de Ca$^{2+}$

| Lactose | Asialofetuine | Melibiose | Mannane | Fucose |
|---------|---------------|-----------|---------|--------|
| 32 | | | | |
| 64 | - | - | - | 140 |

### Indépendantes de Ca$^{2+}$

| Lactose | Asialofetuine | Melibiose | Mannane | Fucose |
|---------|---------------|-----------|---------|--------|
| 22 | - | - | 44 | 13 |
| 52 | | | 46 | 30 |
| | | | | 42 |
| | | | | 45 |
| | | | | 62 |

5. Procédé selon la revendication 1 ou 2, dans lequel les lectines sont caractérisées en ce qu'elles proviennent de teratome murin, ont la masse moléculaire ci-après (en milliers) et se lient spécifiquement aux hydrates de carbone suivants:

### Dépendantes de Ca$^{2+}$

| Lactose | Asialofetuine | Melibiose | Mannane | Fucose |
|---------|---------------|-----------|---------|--------|
| 24 | - | - | 32 | 32 |

### Indépendantes de Ca$^{2+}$

| Lactose | Asialofetuine | Melibiose | Mannane | Fucose |
|---------|---------------|-----------|---------|--------|
| - | - | - | - | $\approx 100$ |

6. Procédé selon la revendication 1 ou 2, dans lequel les lectines sont caractérisées en ce qu'elles proviennent d'une tumeur épithéliale maligne humaine, présentent la masse moléculaire suivante (en milliers) et se lient spécifiquement aux hydrates de carbone suivants:

### Dépendantes de Ca$^{2+}$

| Lactose | Asialofetuine | Melibiose | Mannane | Fucose |
|---------|---------------|-----------|---------|--------|
| 70 | - | 28 | - | 62 |
| | | 43 | | 70 |
| | | 45 | | |

### Indépendantes de Ca$^{2+}$

| Lactose | Asialofetuine | Melibiose | Mannane | Fucose |
|---------|---------------|-----------|---------|--------|
| - | - | 64 | - | 62 |

**7.** Procédé selon la revendication 1 ou 2, dans lequel les lectines sont caractérisées en ce qu'elles proviennent d'un teratocarcinome humain, ont la masse moléculaire suivante (en milliers) et se lient spécifiquement aux hydrates de carbone suivants:

Dépendantes de $Ca^{2+}$

| Lactose | Asialofetuine | Melibiose | Mannane | Fucose |
|---------|---------------|-----------|---------|--------|
| - | - | 31 | - | 31 |
| | | | | 70 |

Indépendantes de $Ca^{2+}$

| Lactose | Asialofetuine | Melibiose | Mannane | Fucose |
|---------|---------------|-----------|---------|--------|
| - | - | - | 68 | - |

**8.** Procédé de production d'un anticorps monoclonal ou d'un sous-fragment de celui-ci spécifiquement dirigé vers une lectine obtenable par un procédé selon l'une quelconque des revendications 1 à 7, ledit procédé consistant à isoler ledit anticorps d'un hybridome humain ou murin et, facultativement, à cliver par voie protéolytique l'anticorps isolé.

**9.** Procédé de production d'une composition de diagnostic pour la détection de cellules tumorales chez les mammifères, caractérisé en ce qu'on conjugue une lectine obtenable selon un procédé de l'une quelconque des revendications 1 à 7, un anticorps monoclonal ou un sous-fragment de celui-ci qu'on obtient par le procédé de la revendication 8, ou un dérivé de ladite lectine ou dudit anticorps monoclonal ou d'un sous-fragment de celui-ci, avec un composé permettant sa détection dans un titrage approprié pour un diagnostic différentiel des types de tumeurs et du stade de développppement des tumeurs.

**10.** Utilisation d'une lectine obtenable par un procédé selon l'une quelconque des revendications 1 à 7, d'un anticorps monoclonal ou d'un sous-fragment de celui-ci qu'on obtient par le procédé de la revendication 8, ou d'un dérivé de ladite lectine ou de l'anticorps pour la production d'une composition de diagnostic servant à la détection de cellules tumorale chez les mammifères.

**11.** Utilisation d'un hydrate de carbone reconnaissable par une lectine qu'on obtient par le procédé selon l'une quelconque des revendications 1 à 7, ou d'un dérivé dudit hydrate de carbone, pour la production d'une composition de diagnostic servant à la détection des cellules tumorales chez les mammifères.

**12.** Procédé de production d'une composition pharmaceutique de traitement de néoplasies malignes par une destruction des cellules tumorales et/ou inhibition de la formation des métastases, qui consiste à combiner une quantité efficace d'une lectine obtenable par un procédé selon l'une quelconque des revendications 1 à 7, un anticorps monoclonal ou un sous-fragment de celui-ci qu'on obtient par le procédé de la revendication 8, ou d'un dérivé de ladite lectine ou de l' anticorps avec un véhicule ou diluant pharmaceutiquement acceptable.

**13.** Utilisation d'un hydrate de carbone reconnaissable par une lectine qu'on obtient par un procédé selon l'une quelconque des revendications 1 à 7, ou d'un dérivé dudit hydrate de carbone pour la production d'une composition pharmaceutique servant au traitement des néoplasies malignes par une destruction spécifique des cellules tumorales et/ou inhibition de la formation des métastases.

**Patentansprüche**
**Patentansprüche für folgende Vertragsstaaten: BE, CH, DE, FR, GB, IT, LI, LU, NL, SE**

**1.** Kohlenhydrat-bindende Proteine (Lectine), erhalten aus einer Säugertumorzelle, die spezifisch auf der Oberfläche von Tumorzellen, aber nicht auf der Oberfläche von normalen Zellen vorkommen.

2. Lectine nach Anspruch 1, dadurch gekennzeichnet, daß sie von einem Ratten-Rhabdomyosarcom abgeleitet sind, das folgende Molekulargewicht (in tausend) haben und spezifisch an die folgenden Kohlenhydrate binden:

$Ca^{2+}$-unabhängig

| Lactose | Asialofetuin | Melibiose | Mannan | Fucose |
|---|---|---|---|---|
| — | — | 29 | 60–72 | 60–72 |
| | | 43 | | |
| | | 45 | | |

3. Lectine nach Anspruch 1, dadurch gekennzeichnet, daß sie von einem invasiven Ratten-Tubulopapillar-Adenocarcinom mit einem niedrigen Differenzierungsgrad abgeleitet sind, das folgende Molekulargewicht (in tausend) haben und spezifisch an die folgenden Kohlenhydrate binden:

$Ca^{2+}$-abhängig

| Lactose | Asialofetuin | Melibiose | Mannan | Fucose |
|---|---|---|---|---|
| 32 | | | | |
| 64 | — | — | — | 140 |

$Ca^{2+}$-unabhängig

| Lactose | Asialofetuin | Melibiose | Mannan | Fucose |
|---|---|---|---|---|
| 22 | — | — | 44 | 13 |
| 52 | | | 46 | 30 |
| | | | | 42 |
| | | | | 45 |
| | | | | 62 |

4. Lectine nach Anspruch 1, dadurch gekennzeichnet, daß sie von einem Maus-Teratom abgeleitet sind, das folgende Molekulargewicht (in tausend) haben und spezifisch an die folgenden Kohlenhydrate binden:

$Ca^{2+}$-abhängig

| Lactose | Asialofetuin | Melibiose | Mannan | Fucose |
|---|---|---|---|---|
| 24 | — | — | 32 | 32 |

$Ca^{2+}$-unabhängig

| Lactose | Asialofetuin | Melibiose | Mannan | Fucose |
|---|---|---|---|---|
| — | — | — | — | ≈100 |

5. Lectine nach Anspruch 1, dadurch gekennzeichnet, daß sie von einem menschlichen malignen Epitheltumor abgeleitet sind, das folgende Molekulargewicht (in tausend) haben und spezifisch an die folgenden Kohlenhydrate binden:

Ca$^{2+}$-abhängig

| Lactose | Asialofetuin | Melibiose | Mannan | Fucose |
|---------|-------------|-----------|--------|--------|
| 70 | - | 28 | - | 62 |
| | | 43 | | 70 |
| | | 45 | | |

Ca$^{2+}$-unabhängig

| Lactose | Asialofetuin | Melibiose | Mannan | Fucose |
|---------|-------------|-----------|--------|--------|
| - | - | 64 | - | 62 |

6. Lectine nach Anspruch 1, dadurch gekennzeichnet, daß sie von einem menschlichen Teratocarcinom abgeleitet sind, das folgende Molekulargewicht (in tausend) haben und spezifisch an die folgenden Kohlenhydrate binden:

Ca$^{2+}$-abhängig

| Lactose | Asialofetuin | Melibiose | Mannan | Fucose |
|---------|-------------|-----------|--------|--------|
| - | - | 31 | - | 31 |
| | | | | 70 |

Ca$^{2+}$-unabhängig

| Lactose | Asialofetuin | Melibiose | Mannan | Fucose |
|---------|-------------|-----------|--------|--------|
| - | - | - | 68 | - |

7. Verfahren zur Herstellung der Lectine nach den Ansprüchen 1 bis 6, das die folgenden Schritte umfaßt:
   - Extraktion des Tumorgewebes mit Aceton;
   - Abdampfen des Acetonextrakts, um ein Acetonpulver zu erhalten;
   - Extraktion des Acetonpulvers mit einer gepufferten wäßrigen Lösung zur Lösung der Lectine;
   - Adsorption der im wäßrigen Extrakt enthaltenen Lectine an Affinitätschromatographie-Säulen, an die Kohlenhydrate gebunden sind, die von den Lectinen erkannt werden können;
   - Elution der Ca$^{2+}$-abhängigen Lectine von der Säule mit einer wäßrigen Lösung eines Chelatbildners; und anschließend
   - Elution der Ca$^{2+}$-unabhängigen Lectine von der Säule mit einer wäßrigen Lösung eines Kohlenhydrats, das mit dem an die Säule gebundenen Kohlenhydrat um die Bindungsstelle des Lectins kompetiert.

8. Verfahren nach Anspruch 7, dadurch gekennzeichnet, daß der wäßrige Extrakt durch Homogenisierung des Tumorgewebes in einem flüssigen Extraktionsmedium und durch Zentrifugation und Dialyse des Homogenisats erhalten wird.

9. Monoclonaler Antikörper oder Subfragment davon, erhalten von einem menschlichen oder einem Maus-Hybridom, dadurch gekennzeichnet, daß er/es spezifisch gegen ein Lectin nach einem der Ansprüche 1 bis 6 gerichtet ist.

10. Diagnostische Zusammensetzung zum Nachweis von Säugertumorzellen, dadurch gekennzeichnet, daß sie ein Lectin nach einem der Ansprüche 1 bis 6, einen monoclonalen Antikörper oder ein Subfragment davon, der/das spezifisch gegen ein Lectin nach einem der Ansprüche 1 bis 6 gerichtet ist, oder ein Derivat des Lectins oder des monoclonalen Antikörpers enthält.

**11.** Verwendung eines Lectins nach einem der Ansprüche 1 bis 6, eines monoclonalen Antikörpers oder eines Subfragments davon, der/das spezifisch gegen ein Lectin nach einem der Ansprüche 1 bis 6 gerichtet ist, oder eines Derivates des Lectins oder des Antikörpers zur Herstellung einer diagnostischen Zusammensetzung zum Nachweis von Säugertumorzellen.

**12.** Verwendung eines durch ein Lectin nach einem der Ansprüche 1 bis 6 erkennbaren Kohlenhydrats oder eines Derivats des Kohlenhydrats zur Herstellung einer diagnostischen Zusammensetzung zum Nachweis von Säugertumorzellen.

**13.** Pharmazeutische Zusammensetzung zur Behandlung maligner Neoplasien durch spezifische Zerstörung von Tumorzellen und/oder Inhibition der Metastasierung, enthaltend eine wirksame Menge eines Lectins nach einem der Ansprüche 1 bis 6, einen monoclonalen Antikörper oder ein Subfragment davon, der/das spezifisch gegen ein Lectin nach einem der Ansprüche 1 bis 6 gerichtet ist, oder ein Derivat des Lectins oder des monoclonalen Antikörpers und einen pharmazeutisch verträglichen Träger oder Verdünnungsmittel.

**14.** Verwendung eines durch ein Lectin nach einem der Ansprüche 1 bis 6 erkennbaren Kohlenhydrats oder eines Derivats des Kohlenhydrats zur Herstellung einer pharmazeutischen Zusammensetzung zur Behandlung maligner Neoplasien durch spezifische Zerstörung von Tumorzellen und/oder Inhibition der Metastasierung.

**Patentansprüche für folgenden Vertragsstaat: AT**

**1.** Verfahren zum Erhalten von Kohlenhydrat-bindenden Proteinen (Lectinen) aus einer Säugertumorzelle, wobei die Lectine spezifisch auf der Oberfläche von Tumorzellen, aber nicht auf der Oberfläche von normalen Zellen vorkommen und wobei das Verfahren die folgenden Schritte umfaßt:
- Extraktion des Tumorgewebes mit Aceton;
- Abdampfen des Acetonextrakts, um ein Acetonpulver zu erhalten;
- Extraktion des Acetonpulvers mit einer gepufferten wäßrigen Lösung zur Lösung der Lectine;
- Adsorption der im wäßrigen Extrakt enthaltenen Lectine an Affinitätschromatographie-Säulen, an die Kohlenhydrate gebunden sind, die von den Lectinen erkannt werden können;
- Elution der $Ca^{2+}$-abhängigen Lectine von der Säule mit einer wäßrigen Lösung eines Chelatbildners; und anschließend
- Elution der $Ca^{2+}$-unabhängigen Lectine von der Säule mit einer wäßrigen Lösung eines Kohlenhydrats, das mit dem an die Säule gebundenen Kohlenhydrat um die Bindungsstelle des Lectins kompetiert.

**2.** Verfahren nach Anspruch 1, dadurch gekennzeichnet, daß der wäßrige Extrakt durch Homogenisierung des Tumorgewebes in einem flüssigen Extraktionsmedium und durch Zentrifugation und Dialyse des Homogenisats erhalten wird.

**3.** Verfahren nach Anspruch 1 oder 2, wobei die Lectine dadurch gekennzeichnet sind, daß sie von einem Ratten-Rhabdomyosarcom abgeleitet sind, das folgende Molekulargewicht (in tausend) haben und spezifisch an die folgenden Kohlenhydrate binden:

$Ca^{2+}$-unabhängig

| Lactose | Asialofetuin | Melibiose | Mannan | Fucose |
|---------|-------------|-----------|--------|--------|
| - | - | 29<br>43<br>45 | 60-72 | 60-72 |

**4.** Verfahren nach Anspruch 1 oder 2, wobei die Lectine dadurch gekennzeichnet sind, daß sie von einem invasiven Ratten-Tubulopapillar-Adenocarcinom mit einem niedrigen Differenzierungsgrad abgeleitet sind, das folgende Molekulargewicht (in tausend) haben und spezifisch an die folgenden Kohlenhydrate

binden:

$Ca^{2+}$-abhängig

| Lactose | Asialofetuin | Melibiose | Mannan | Fucose |
|---|---|---|---|---|
| 32 | | | | |
| 64 | — | — | — | 140 |

$Ca^{2+}$-unabhängig

| Lactose | Asialofetuin | Melibiose | Mannan | Fucose |
|---|---|---|---|---|
| 22 | — | — | 44 | 13 |
| 52 | | | 46 | 30 |
| | | | | 42 |
| | | | | 45 |
| | | | | 62 |

5. Verfahren nach Anspruch 1 oder 2, wobei die Lectine dadurch gekennzeichnet sind, daß sie von einem Maus-Teratom abgeleitet sind, das folgende Molekulargewicht (in tausend) haben und spezifisch an die folgenden Kohlenhydrate binden:

$Ca^{2+}$-abhängig

| Lactose | Asialofetuin | Melibiose | Mannan | Fucose |
|---|---|---|---|---|
| 24 | — | — | 32 | 32 |

$Ca^{2+}$-unabhängig

| Lactose | Asialofetuin | Melibiose | Mannan | Fucose |
|---|---|---|---|---|
| — | — | — | — | $\approx 100$ |

6. Verfahren nach Anspruch 1 oder 2, wobei die Lectine dadurch gekennzeichnet sind, daß sie von einem menschlichen malignen Epitheltumor abgeleitet sind, das folgende Molekulargewicht (in tausend) haben und spezifisch an die folgenden Kohlenhydrate binden:

$Ca^{2+}$-abhängig

| Lactose | Asialofetuin | Melibiose | Mannan | Fucose |
|---|---|---|---|---|
| 70 | — | 28 | — | 62 |
| | | 43 | | 70 |
| | | 45 | | |

$Ca^{2+}$-unabhängig

| Lactose | Asialofetuin | Melibiose | Mannan | Fucose |
|---|---|---|---|---|
| — | — | 64 | — | 62 |

7. Verfahren nach Anspruch 1 oder 2, wobei die Lectine dadurch gekennzeichnet sind, daß sie von einem menschlichen Teratocarcinom abgeleitet sind, das folgende Molekulargewicht (in tausend) haben und spezifisch an die folgenden Kohlenhydrate binden:

$Ca^{2+}$-abhängig

| Lactose | Asialofetuin | Melibiose | Mannan | Fucose |
|---------|--------------|-----------|--------|--------|
| - | - | 31 | - | 31 70 |

$Ca^{2+}$-unabhängig

| Lactose | Asialofetuin | Melibiose | Mannan | Fucose |
|---------|--------------|-----------|--------|--------|
| - | - | - | 68 | - |

8. Verfahren zur Herstellung eines monoclonalen Antikörpers oder eines Subfragments davon, der/das spezifisch gegen ein nach einem Verfahren nach einem der Ansprüche 1 bis 7 erhältliches Lectin gerichtet ist, wobei das Verfahren die Isolierung des Antikörpers aus einem menschlichen oder Maus-Hybridom umfaßt und gegebenenfalls die proteolytische Spaltung des isolierten Antikörpers.

9. Verfahren zur Herstellung einer diagnostischen Zusammensetzung zum Nachweis von Säugertumorzellen, dadurch gekennzeichnet, daß ein nach einem Verfahren nach einem der Ansprüche 1 bis 7 erhältliches Lectin, ein monoclonaler Antikörper oder ein Subfragment davon, erhältlich nach dem Verfahren von Anspruch 8, oder ein Derivat des Lectins oder des monoclonalen Antikörpers oder Subfragments davon mit einer Verbindung konjugiert wird, die dessen Nachweis in einem geeigneten Test zur differentiellen Diagnose von Tumortypen und dem Entwicklungsstadium von Tumoren gestattet.

10. Verwendung eines Lectins, das nach einem Verfahren nach einem der Ansprüche 1 bis 7 erhältlich ist, eines monoclonalen Antikörpers oder eines Subfragments davon, der/das nach dem Verfahren von Anspruch 8 erhältlich ist, oder eines Derivats des Lectins oder Antikörpers zur Herstellung einer diagnostischen Zusammensetzung zum Nachweis von Säugertumorzellen.

11. Verwendung eines Kohlenhydrats, das durch ein nach einem Verfahren nach einem der Ansprüche 1 bis 7 erhältlichen Lectin erkennbar ist, oder eines Derivats des Kohlenhydrats zur Herstellung einer diagnostischen Zusammensetzung zum Nachweis von Säugertumorzellen.

12. Verfahren zur Herstellung einer pharmazeutischen Zusammensetzung zur Behandlung maligner Neoplasien durch die spezifische Zerstörung von Tumorzellen und/oder Inhibition der Metastasierung, umfassend das Vermischen einer wirksamen Menge eines nach einem Verfahren nach einem der Ansprüche 1 bis 7 erhältlichen Lectins, eines monoclonalen Antikörpers oder eines Subfragments davon, erhältlich nach dem Verfahren von Anspruch 8, oder eines Derivats des Lectins oder Antikörpers mit einem pharmazeutisch verträglichen Träger oder Verdünnungsmittel.

13. Verwendung eines Kohlenhydrats, das durch ein nach einem Verfahren nach einem der Ansprüche 1 bis 7 erhältliches Lectin erkennbar ist, oder eines Derivats des Kohlenhydrats zur Herstellung einer pharmazeutischen Zusammensetzung zur Behandlung maligner Neoplasien durch spezifische Zerstörung von Tumorzellen und/oder Inhibition der Metastasierung.

Figure 1

A

Heterotypic aggregation

B

Sugar

Lectin

Homotypic aggregation

Figure 2